# EUROPÄISCHE PATENTSCHRIFT

(11) **EP 4 103 212 B1**
(45) Veröffentlichungstag und Bekanntmachung des Hinweises auf die Patenterteilung: **08.07.2026**
(21) Anmeldenummer: 20806922.9
(22) Anmeldetag: 06.11.2020
(51) Int. Cl.: A61K 36/534, A61K 36/61, A61K 36/63, A61K 36/752, A61P 31/14, A61P 11/00, A61P 31/12, A61K 8/92

(54) **ZUSAMMENSETZUNG UMFASSEND OLIVENÖL, ZITRONENÖL, PFEFFERMINZÖL, EUKALYPTUSÖL UND LAVENDELÖL ZUR VERWENDUNG IN DER BEHANDLUNG VON NASENERKRANKUNGEN**
COMPOSITION INCLUDING OLIVE OIL, LEMON OIL, PEPPERMINT OIL, EUCALYPTUS OIL AND LAVENDER OIL FOR USE IN THE TREATMENT OF NASAL DISEASES
COMPOSITION À BASE D'HUILE D'OLIVE, D'HUILE DE CITRON, D'HUILE DE MENTHE POIVRÉE, D'HUILE D'EUCALYPTUS ET D'HUILE DE LAVANDE, POUR L'UTILISATION DANS LE TRAITEMENT DES MALADIES NASALES

(30) Priorität: 12.02.2020 AT 5002820 U
(43) Veröffentlichungstag der Anmeldung: 21.12.2022
(73) Patentinhaber: Bentsen, Paal, Wien 1090 (AT)
(72) Erfinder: Bentsen, Paal, Wien 1090 (AT)
(74) Vertreter: Schwarz & Partner Patentanwälte GmbH
(86) Internationale Anmeldenummer: PCT/AT2020/060394
(87) Internationale Veröffentlichungsnummer: WO 2021/159155

(56) Entgegenhaltungen:
- EP-A1- 2 801 263
- WO-A1-2012/110672
- WO-A1-2017/023162
- CN-A- 103 070 918
- DE-A1- 102010 054 461
- US-A1- 2008 031 831
- US-A1- 2009 191 249

## Beschreibung

### TECHNISCHES GEBIET

Die Erfindung betrifft eine Zusammensetzung auf Ölbasis zur nasalen Anwendung.

### HINTERGRUND DER ERFINDUNG

Die Haut des menschlichen Körpers kann grob in zwei Gruppen unterteilt werden: die äußere Haut (als Hüllorgan zur Abgrenzung nach außen) und die innere Haut (hauptsächlich zur Abgrenzung von Hohlorganen). Die innere Haut wird auch als Schleimhaut bezeichnet.

Schleimhäute haben meist keinen direkten Kontakt zur Außenwelt. Eine Ausnahme hiervon stellt die Nasenschleimhaut dar.

Da die Nasenschleimhaut nicht dauerhaft physisch von der Außenwelt abgrenzbar ist, ist sie im Vergleich zu nach außen hin abgeschlossenen Schleimhäuten, wie etwa der Magen- oder Darmschleimhaut, Umwelteinflüssen direkt exponiert. In einem gesunden Individuum mit intakter Nasenschleimhaut stellen die üblichen Umwelteinflüsse in der Regel kein großes Problem dar. Kann die Nasenschleimhaut jedoch ihre Barrierefunktion, z.B. durch Verletzungen, nicht wahrnehmen kann dies zu einer deutlich geringeren Resistenz gegenüber Umwelteinflüssen führen, wodurch pathogene Keime oder Viren einfacher vom Körper aufgenommen werden können.

Nasenerkrankungen wie Entzündungen der Nasenschleimhaut können beispielsweise durch eine trockene Umgebungsluft entstehen oder zumindest begünstigt werden. Weiters können Wunden in der Nasenschleimhaut auch auf Operationen der Nase zurückzuführen sein.

Eine rasche Heilung einer Nasenerkrankung ist erstrebenswert, da sich die Heilung der Nasenerkrankung aufgrund ihrer exponierten Lage und den damit einhergehenden Umwelteinflüssen verzögern kann bzw. im schlimmsten Fall das Ausmaß der Erkrankung sogar zunimmt.

Nasenerkrankungen können zu einer Vielzahl an Reaktionen innerhalb der Nase führen. Oftmals ist jedoch ein Anschwellen der Nasenschleimhaut das Resultat. Daher stellen aktuelle Behandlungsmöglichkeiten von Nasenerkrankungen vielfach Schleimhaut-abschwellende Präparate dar, die beispielsweise Xylometazolin als Wirkstoff umfassen können.

Die Anwendung von Xylometazolin und ähnlichen Wirkstoffen kann jedoch bereits nach kurzer, und auf diesem Gebiet durchaus üblichen, Anwendungsdauer von einigen Tagen zur Toleranzentwicklung oder gar Abhängigkeit. Aufgrund der Tatsache, dass Medikamente, die diese Wirkstoffe umfassen, üblicherweise Wasser als Trägerstoff umfassen, kann es durch Verdampfung des Wassers rasch zu Trockenheit und Risse in der Nasenschleimhaut kommen, wodurch bleibende Schäden nicht ausgeschlossen sind.
In der DE 10 2010 054 461 werden wässrige Badezubereitungen offenbart, welche u.a. Lipide (wie z.B. Olivenöl) und ätherische Öle umfassen.
Die EP 2 801 263 beschreibt kühlende Zusammensetzungen, welche u.a. Öle umfassen können. Diese Zusammensetzungen werden für Nahrungs- und Genussmittel, wie Kaugummi und Bonbons, eingesetzt.
In der US 2009/0191249 werden mit ätherischen Ölen versetzte Artikel beschrieben. Diese Artikel können zur Behandlung viraler Erkrankungen eingesetzt werden.
In der CN 103 070 918 wird ein Körperöl beschrieben, welches zum Entfernen von humanem Papillomavirus (HPV) von der Haut geeignet ist.
Die WO 2012/110672 betrifft pharmazeutische Zusammensetzungen, die Aciclovir und Olivenöl umfassen.
In US 2008/031831 werden oral zu verabreichende Zusammensetzungen offenbart, die Öle unterschiedlicher Art umfassen können.
Die WO 2017/023162 A1 beschreibt eine Zusammensetzung, die Olivenöl und ätherische Öle wie beispielsweise Pfefferminzöl, Eukalyptusöl, Lavendelöl und Zitronenöl umfasst. Diese kann nasal verabreicht und zur Behandlung von Allergien verwendet werden.

Daher ist es eine Aufgabe der vorliegenden Erfindung eine Zusammensetzung bereitzustellen, die u.a. zur Pflege von Nasenschleimhäuten und in der Behandlung von Nasenerkrankungen verwendet werden kann und die die Nachteile des Standes der Technik nicht aufweist.

### ZUSAMMENFASSUNG DER ERFINDUNG

Es wurde überraschenderweise festgestellt, dass eine Zusammensetzung umfassend 0,50 Gew% bis 1,00 Gew% Zitronenöl, 0,30 Gew% bis 0,80 Gew% Pfefferminzöl, 0,20 Gew% bis 0,65 Gew% Eukalyptusöl, 0,10 Gew% bis 0,60 Gew% Lavendelöl und Olivenöl bis insgesamt 100 Gew% in der Lage ist, u.a. Reizungen und Erkrankungen der Nasenschleimhäute ausgewählt aus der Gruppe bestehend aus einer Entzündung und einer Wunde, zu behandeln bzw. vorzubeugen wie in den Ansprüchen definiert. Die Kombination von vier der genannten ätherischen Öle zeigt besonders vorteilhafte Wirkungen auf Nasenschleimhäuten, da diese nicht nur zu einer Reduktion von Entzündungen führt, sondern auch die Wundheilung signifikant begünstigt. Durch die Anwendung der erfindungsgemäßen Zusammensetzung können somit sowohl die Symptome von Nasenerkrankungen ausgewählt aus der Gruppe bestehend aus einer Entzündung und einer Wunde, als auch teilweise deren Ursache behandelt werden. Durch das Vorhandensein von Öl in der erfindungsgemäßen Zusammensetzung können zudem trockene Stellen der Nasenschleimhaut "befeuchtet" werden, wodurch die Barrierefunktion der Haut wiederhergestellt werden kann.

### BESCHREIBUNG DER AUSFÜHRUNGSFORMEN

Die erfindungsgemäße Zusammensetzung umfasst 0,50 Gew% bis 1,00 Gew% Zitronenöl, 0,30 Gew% bis 0,80 Gew% Pfefferminzöl, 0,20 Gew% bis 0,65 Gew% Eukalyptusöl, 0,10 Gew% bis 0,60 Gew% Lavendelöl und Olivenöl bis insgesamt 100 Gew%.

"Ätherische Öle", wie hier verwendet, sind leicht flüchtige Stoffgemische, welche aus natürlichen Quellen durch Wasserdampfdestillation, Extraktion oder mechanische Verfahren wie Auspressen von Pflanzen oder Pflanzenteilen gewonnen werden. Zitronenöl wird vorzugsweise aus Zitronenschalen, Pfefferminzöl vorzugsweise aus Pfefferminzblätter, Eukalyptusöl vorzugsweise aus Eukalyptusblätter und Eukalyptuszweigen und Lavendelöl vorzugsweise aus Lavendelblüten gewonnen.

Die Gewinnung von ätherischen Ölen, die in der erfindungsgemäßen Zusammensetzung verwendet werden, kann beispielsweise mittels Wasserdampfdestillation, Kaltpressung, CO₂-Extraktion, Extraktion unter Einsatz von Lösungsmitteln oder Enfleurage erfolgen, wobei Zitronenöl vorzugsweise durch Auspressen von Zitronenschalen und Lavendelöl, Eukalyptusöl und Pfefferminzöl durch Wasserdampfdestillation gewonnen wird. Bei der Herstellung von Pfefferminzöl wird üblicherweise Menthofuran durch Destillation abgetrennt, da dieses eine toxische Wirkung auf den menschlichen und tierischen Körper haben kann.

Das in der erfindungsgemäßen Zusammensetzung vorhandene Olivenöl kann entweder durch Warm- oder Kaltpressverfahren hergestellt werden. Daher ist das verwendete Olivenöl gemäß einer bevorzugten Ausführungsform der vorliegenden Erfindung kaltgepresstes oder warmgepresstes Olivenöl.

"Kaltgepresst", wie hier verwendet, bedeutet, dass die Extraktion des Olivenöls ohne Zufuhr von Wärme stattfindet. Kaltgepresste Olivenöle zeichnen sich durch einen natürlichen Geschmack und den Erhalt von wertvollen Inhaltsstoffen, wie etwa Vitaminen und ungesättigten Fettsäuren aus. Ein Nachteil ist, dass kaltgepresstes Olivenöl bei späterer Erhitzung leichter instabil wird und bei starker Erhitzung sogar gesundheitsschädliche Abbauprodukte bilden kann. Zudem hat kaltgepresstes Olivenöl ein starkes Eigenaroma, welches mitunter unerwünscht ist. Ein weiterer Nachteil von kaltgepressten Olivenöl ist die geringe Lagerstabilität und die Neigung zu Oxidation. Bei kurzen Lagerzeiten spielt diese Eigenschaft keine entscheidende Rolle. Bei Produkten, die eine Lagerzeit von mehr als 6 Monaten, vorzugsweise von mehr als 12 Monaten, haben sollen, ist es von Vorteil warmgepresstes Olivenöl zu verwenden. Bei kaltgepressten Olivenöl kann es bei geringen Lagertemperaturen (z.B. unter 10°C, vorzugsweise unter 5°C) zu Ausflockungen kommen, die ebenfalls unerwünscht sind.

"Warmgepresst", wie hier verwendet, bedeutet, dass die Extraktion des Olivenöls unter Zufuhr von Wärme stattfindet. Warmgepresstes Olivenöl ist im Gegensatz zu kaltgepresstem Olivenöl deutlich unempfindlicher gegenüber Wärme, was u.a. die Lagerung von daraus hergestellten Produkten erleichtert.

Gemäß einer weiteren bevorzugten Ausführungsform ist das Olivenöl raffiniert.

"Raffiniert", wie hier verwendet, bedeutet, dass ein warmgepresstes Olivenöl weiterverarbeitet wird. Durch diesen weiteren Schritt der Raffination werden unter anderem Trübstoffe, aber auch Fettsäuren und andere Inhaltsstoffe, wie etwas Vitamine, abgetrennt. Auch gesundheitsschädliche und geschmacksbeeinträchtigende Inhaltsstoffe können im Zuge der Raffination entfernt werden. Dadurch wird das Öl fast geruchsfrei und geschmacksneutral.

Raffiniertes Olivenöl ist im Vergleich zu warmgepresstem Olivenöl wärmestabiler und lagerfähiger. Diese Eigenschaften sind insbesondere bei der Verwendung der erfindungsgemäßen Zusammensetzung als Medikament vorteilhaft, da Medikamente strengen Auflagen unterliegen und diese durch eine höhere Wärmestabilität und Lagerfähigkeit leichter erfüllt werden können.

Gemäß einer bevorzugten Ausführungsform ist das Zitronenöl ein Zitronenöl von *Citrus x limon.*

Gemäß einer bevorzugten Ausführungsform ist das Pfefferminzöl ein Pfefferminzöl von *Mentha x piperita.*

Gemäß einer bevorzugten Ausführungsform ist das Eukalyptusöl ein Eukalyptusöl von *Eucalyptus globulus.*

Gemäß einer bevorzugten Ausführungsform ist das Lavendelöl ein Lavendelöl von *Lavandula angustifolia.*

Bei ätherischen Ölen handelt es sich um Gemische verschiedener Stoffe und Wirkstoffe aus den jeweiligen Pflanzen. Da diese Inhaltsstoffe unterschiedliche Wirkungen haben können, kann es von Vorteil sein, das Verhältnis zwischen den eingesetzten ätherischen Ölen zu variieren, um den gewünschten oder um einen verbesserten Effekt zu erzielen.

Gemäß einer bevorzugten Ausführungsform beträgt die Konzentration des Zitronenöls in der Zusammensetzung 0,60 Gew% bis 1,00 Gew%, bevorzugt 0,70 Gew% bis 1,00 Gew%, noch bevorzugter 0,80 Gew% bis 1,00 Gew%, noch bevorzugter 0,90 Gew% bis 1,00 Gew%, noch bevorzugter 0,90 Gew% bis 0,95 Gew%, insbesondere 0,95 Gew%.

Gemäß einer bevorzugten Ausführungsform beträgt die Konzentration des Pfefferminzöls in der Zusammensetzung 0,40 Gew% bis 0,80 Gew%, bevorzugt 0,50 Gew% bis 0,80 Gew%, noch bevorzugter 0,60 Gew% bis 0,80 Gew%, noch bevorzugter 0,70 Gew% bis 0,80 Gew%, noch bevorzugter 0,70 Gew% bis 0,75 Gew%, insbesondere 0,75 Gew%.

Gemäß einer weiteren bevorzugten Ausführungsform beträgt die Konzentration des Eukalyptusöls in der Zusammensetzung 0,30 Gew% bis 0,65 Gew%, bevorzugt 0,40 Gew% bis 0,65 Gew%, noch bevorzugter 0,50 Gew% bis 0,65 Gew%, noch bevorzugter 0,5 Gew% bis 0,60 Gew%, insbesondere 0,5 oder 0,60 Gew%.

Gemäß einer bevorzugten Ausführungsform beträgt die Konzentration des Lavendelöls in der Zusammensetzung 0,20 Gew% bis 0,60 Gew%, bevorzugt 0,30 Gew% bis 0,60 Gew%, noch bevorzugter 0,40 Gew% bis 0,60 Gew%, noch bevorzugter 0,50 Gew% bis 0,60 Gew%, noch bevorzugter 0,50 Gew% bis 0,55 Gew%, insbesondere 0,55 Gew%.

Es hat sich erfindungsgemäß gezeigt, dass diese Konzentrationen an ätherischen Ölen besonders vorteilhaft bei der Anwendung zur Behandlung von Erkrankungen betreffend die Nasenschleimhaut sind.

Die Gesamtmenge an ätherischen Ölen in der erfindungsgemäßen Zusammensetzung kann somit vorzugsweise 1,1 Gew% bis 3,05 Gew% betragen, sofern die erfindungsgemäße Zusammensetzung Zitronenöl, Pfefferminzöl, Eukalyptusöl und Lavendelöl umfasst. Olivenöl wird zu den mindestens drei ätherischen Ölen hinzugegeben bis insgesamt 100 Gew% erreicht wird.

Besonders vorteilhaft ist eine Zusammensetzung, welche 0,90 Gew% bis 0,95 Gew%, Zitronenöl, 0,70 Gew% bis 0,75 Gew%, Pfefferminzöl, 0,5 oder 0,60 Gew%, Eukalyptusöl und 0,50 Gew% bis 0,55 Gew%, Lavendelöl umfasst.

Die erfindungsgemäße Zusammensetzung kann als Nasenöl verwendet werden.

Hierfür eignet sich die erfindungsgemäße Zusammensetzung aufgrund ihrer öligen Bestandteile gut. Die erfindungsgemäße Zusammensetzung kann einen öligen Film auf der Haut, wie der Schleimhaut der Nase, ausbilden. Dieser kann nach einer gewissen Zeit, und abhängig von der aufgetragenen Menge, von der Haut aufgenommen werden.

Die erfindungsgemäß eingesetzten ätherischen Öle sind jedoch leicht flüchtige Stoffe und können daher, zumindest teilweise, aus der erfindungsgemäße Zusammensetzung entweichen. Durch die Verwendung der erfindungsgemäßen Zusammensetzung als Nasenöl und dem daraus folgenden Einsatz in der Nase, werden die flüchtigen ätherischen Öle durch die Atmung im gesamten Nasen-, Nasennebenhöhlen- und Rachenraum, sowie in der Luftröhre, den Bronchien und den Lungenbläschen verteilt. Daher können die ätherischen Öle bei Verwendung des erfindungsgemäßen Nasenöles ihre Wirkung nicht nur lokal durch Absorption, sondern auch durch den Transport durch den Atem entfalten.

Die erfindungsgemäße Zusammensetzung wird zur Behandlung von Nasenerkrankungen ausgewählt aus der Gruppe bestehend aus einer Entzündung und einer Wunde verwendet.

Wie oben ausgeführt, weist die erfindungsgemäße Zusammensetzung, vielseitige Wirkungen auf. Beispielsweise hat sich gezeigt, dass diese Zusammensetzung entzündungshemmend, krampflösend, antimikrobiell, schmerzstillend, antiviral, juckreizstillend und schleimlösend ist.

Es hat sich gezeigt, dass diese Wirkungen bei der Behandlung verschiedenste Nasenerkrankungen vorteilhaft sein können. Einerseits kann die Ursache einer Nasenerkrankung durch beispielsweise die antivirale Wirkung bekämpft werden. Andererseits können Symptome beispielsweise durch die entzündungshemmende Wirkung bekämpft werden und so dem erkrankten Individuum Linderung verschaffen.

Die erfindungsgemäße Zusammensetzung zeigt vor allem gegenüber Viren eine besonders starke Wirksamkeit. Es ist mit der erfindungsgemäßen Zusammensetzung möglich, Viren zu inaktivieren bzw. deren Infektiosität signifikant zu reduzieren, so dass sich die Zusammensetzung durch Verabreichung an Menschen zur Prävention von viralen Krankheiten einsetzen lässt. Da die erfindungsgemäße Zusammensetzung nasal verabreicht wird, können damit effektiv Krankheiten verhindert bzw. behandelt werden, welche von Viren verursacht werden, die der Mensch über die Schleimhäute, insbesondere über die nasalen Schleimhäute, aufnimmt. Durch das Aufbringen der erfindungsgemäßen Zusammensetzung bildet sich ein Film an der Oberfläche der Schleimhäute, der antivirale Eigenschaften aufweist.

Die erfindungsgemäße Zusammensetzung zeigt insbesondere bei Viren, welche eine Hülle und/oder ein Kapsid umfassen, gute Wirksamkeit. Viren umfassen üblicherweise neben Nukleinsäuremolekülen (DNA oder RNA) Proteine (sog. Kapsidproteine), die das sogenannte Kapsid ausbilden. Zudem können Viren Hüllen aufweisen, die Lipidmembranen und gegebenenfalls darin eingebettete Proteine umfassen. Sowohl das Kapsid als auch die Hülle dienen u.a. zum Schutz der darin befindlichen viralen Nukleinsäuremoleküle. Ohne diesen Schutz sind Viren in aller Regel nicht übertragbar bzw. nicht infektiös. Die erfindungsgemäße Zusammensetzung ist in der Lage, das Kapsid bzw. die Hülle von Viren derart zu beeinflussen, dass sich das Kapsid bzw. die Hülle "auflöst". Demgemäß betrifft ein weiterer Aspekt der vorliegenden Erfindung eine erfindungsgemäße Zusammensetzung zur Verwendung zur Prävention oder Behandlung einer viralen Erkrankung.

Die erfindungsgemäße Zusammensetzung wird zur Prävention oder Inaktivierung von Viren, vorzugsweise von mit einer Hülle ummantelten Viren, eingesetzt.

Gemäß einer besonders bevorzugten Ausführungsform der vorliegenden Erfindung wird die erfindungsgemäße Zusammensetzung zur Prävention und/oder Behandlung von Viren der Familie der Poxviridae, vorzugsweise der Gattung Orthopoxvirus, insbesondere Orthopoxvirus variola, der Familie der Herpesviridae, insbesondere Herpes-simplex-Virus 1 (HSV-1), Herpes-simplex-Virus 2 (HSV-2), Varizella-Zoster-Virus (VZV), humanes Cytomegalievirus (HCMV), humanes Herpesvirus 6 (HHV-6), humanes Herpesvirus 7 (HHV-7) oder Epstein-Barr-Virus (EBV), der Familie der Hepadnaviridae, insbesondere Hepatitis-B-Virus (HBV), der Familie der Familie Flaviviridae, insbesondere Hepatitis-C-Virus (HCV), West-Nil-Virus (WNV), Dengue-Virus (DENV), Gelbfieber-Virus (YFV), Japan-Enzephalitis-Virus (JEV), FSME-Virus oder Zika-Virus (ZIKV), der Familie der Coronaviridae, insbesondere humanes Coronavirus 229E, humanes Coronavirus NL63, SARS-assoziiertes Coronavirus (SARS-CoV) oder SARS-assoziiertes Coronavirus Subtyp SARS-CoV-2, der Familie der Orthomyxoviridae, insbesondere Influenzavirus A-Variante H1N1, Influenzavirus A-Variante H3N2, Influenzavirus-A-Variante H5N1, Influenzavirus B/Victoria-Linie oder Influenzavirus B/Yamagata-Linie, und/oder der Familie der Picornaviridae, insbesondere humane Rhinoviren-1 A oder B. Besonders bevorzugt wird die erfindungsgemäße Zusammensetzung zur Prävention und/oder Behandlung von Viren der Familie der Coronaviridae, insbesondere humanes Coronavirus 229E, humanes Coronavirus NL63, SARS-assoziiertes Coronavirus (SARS-CoV) oder SARS-assoziiertes Coronavirus Subtyp SARS-CoV-2, besonders bevorzugt von SARS-assoziiertem Coronavirus (SARS-CoV) oder SARS-assoziiertem Coronavirus Subtyp SARS-CoV-2, und/oder der Familie der Picornaviridae, insbesondere humane Rhinoviren-1 A oder B.

Offenbart ist auch, dass die Nasenerkrankung eine oberflächliche Irritation der Nasenschleimhaut, bevorzugt eine trockenen Nasenschleimhaut sein kann.

"Oberflächliche Irritation", wie hier verwendet, bedeutet, dass das schützende Deckgewebe intakt ist.

Eine oberflächliche Irritation der Nasenschleimhaut ist bevorzugt eine trockene Nasenschleimhaut.

Da bei oberflächlichen Irritationen das schützende Deckgewebe intakt ist, stellt diese Art der Nasenerkrankungen in der Regel eine leichtere Erkrankung dar. Dies ist unter anderem durch die intakte erste Barriere gegenüber Schädlingen, wie etwa Bakterien, oder physischen Fremdkörpern, wie Staub, zurückzuführen.

Oberflächliche Irritationen der Nasenschleimhaut können vom menschlichen Körper mit nicht allzu großem Aufwand wieder geheilt werden. Beispielsweise reicht im Falle einer trockenen Nasenschleimhaut eine Befeuchtung aus.

"Entzündung", wie hier verwendet, zeichnet sich durch mindestens ein Symptom, bevorzugt zwei Symptome, bevorzugter drei Symptome, noch bevorzugter vier Symptome, insbesondere alle fünf Symptome, ausgewählt aus der Gruppe bestehend aus Rötung, Schwellung, Überwärmung, Schmerz und funktionelle Einschränkung aus.

Je nach Schweregrad der Entzündung ist deren Behebung durch den Körper mit unterschiedlichem Aufwand verbunden.

"Wunde", wie hier verwendet, bedeutet, dass das schützende Deckgewebe der Schleimhaut nicht intakt ist und durchtrennt wurde.

Durch die Durchtrennung der ersten Schutzbarriere ist eine Wunde in der Regel als schwerwiegendere Erkrankung einzustufen. Schädlingen, wie etwa Bakterien, oder physischen Fremdkörpern, wie Staub, können leichter in den Körper eindringen.

Für die Heilung einer Wunde benötigt der Körper mehr Zeit und Aufwand. Es müssen unter anderem neue Zellen gebildet werden und Abfallstoffe, sowie abgestorbene Zellen abtransportiert werden.

Gemäß einer weiteren bevorzugten Ausführungsform ist die Ursache der Nasenerkrankung ausgewählt aus der Gruppe bestehend aus einer trockenen Umgebungsluft, einem erhöhten Naseninnendruck, einer Entzündung, bevorzugt Nasennebenhöhlenentzündung, einer Allergie, einer anderen Erkrankung mit Auswirkungen auf die Nase, bevorzugt einem grippalen Infekt, einem physischen Fremdkörper, einer äußerlichen Gewalteinwirkung und einer Operation.

Eine oberflächliche Irritation der Nasenschleimhaut wird bevorzugt durch eine trockene Umgebungsluft ausgelöst.

Eine Entzündung der Nasenschleimhaut wird bevorzugt durch eine Allergie, eine andere Erkrankung mit Auswirkungen auf die Nase, bevorzugt einem grippalen Infekt, ausgelöst.

Eine Wunde der Nasenschleimhaut wird bevorzugt durch einen erhöhten Naseninnendruck, einen physischen Fremdkörper, eine äußerliche Gewalteinwirkung und eine Operation ausgelöst.

Die erfindungsgemäße Zusammensetzung wird nasal verabreicht.

Der Vorteil einer nasalen Verabreichung der erfindungsgemäßen Zusammensetzung ist einerseits die lokale Absorption der Bestandteile und die Bildung eines vorübergehenden Schutzfilmes auf der Nasenschleimhaut und andererseits die Verteilung der flüchtigen ätherischen Öle durch die Atmung im gesamten Nasen-, Nasennebenhöhlen- und Rachenraum, sowie in der Luftröhre, den Bronchien und den Lungenbläschen.

Gemäß einer weiteren bevorzugten Ausführungsform wird die erfindungsgemäße Zusammensetzung ein- bis mehrmals täglich, bevorzugt ein- bis dreimal, verabreicht.

Das Intervall und damit die Anzahl der Verabreichung über den Tag verteilt können unter anderem von der Art und Schwere der Nasenerkrankung abhängen.

Bei einer leichteren Nasenerkrankung, wie etwa eine oberflächliche Irritation der Nasenschleimhaut, kann eine Verabreichung bis zu dreimal täglich, bevorzugt zweimal täglich, noch bevorzugter einmal täglich, ausreichend sein.

Zur Behandlung einer schwereren Nasenerkrankung, wie beispielsweise einer Wunde, insbesondere einer Wunde nach einer Operation, kann eine Verabreichung von mindestens zweimal täglich, bevorzugt dreimal täglich, notwendig sein.

Gemäß einer bevorzugten Ausführungsform wird die erfindungsgemäße Zusammensetzung über einen Zeitraum von bis zu 3 Monaten, bevorzugt bis zu 2 Monaten, bevorzugter bis zu 1 Monat, noch bevorzugter bis zu 3 Wochen, noch bevorzugter bis zu 2 Wochen, noch bevorzugter bis zu 1 Woche, verabreicht.

Die Dauer der Verabreichung der erfindungsgemäßen Zusammensetzung kann unter anderem von der Art und Schwere der Nasenerkrankung abhängen.

Bei einer leichteren Nasenerkrankung, wie etwa eine oberflächliche Irritation der Nasenschleimhaut, kann eine Verabreichung bis zu 3 Wochen, bevorzugt bis zu 2 Wochen, bevorzugter bis zu 1 Woche, ausreichend sein.

Zur Behandlung einer schwereren Nasenerkrankung, wie beispielsweise einer Wunde, insbesondere einer Wunde nach einer Operation, kann eine Verabreichung von 3 Monaten, bevorzugt bis zu 2 Monaten, bevorzugter bis zu 1 Monat, noch bevorzugter bis zu 3 Wochen, noch bevorzugter bis zu 2 Wochen, noch bevorzugter bis zu 1 Woche, notwendig sein.

Die Verabreichung der erfindungsgemäßen Zusammensetzung kann über einen längeren Zeitraum, bevorzugt von bis zu 3 Monaten, erfolgen. Dabei besteht, im Gegensatz zu anderen üblichen Präparaten auf dem Gebiet, die etwa Xylometazolin als Wirkstoff umfassen, nicht die Gefahr einer Abhängigkeit oder sogar der Entstehung von Langzeitschäden.

Daher eignet sich die erfindungsgemäße Zusammensetzung insbesondere zur Verabreichung über einen längeren Zeitraum von bis zu 3 Monaten. Dies ist besonders bei der Nachbetreuung von Wunden nach einer Operation, wie beispielsweise einer Nasenscheidewandoperation, von Vorteil.

Daher wird die erfindungsgemäße Zusammensetzung insbesondere zur Behandlung von Wunden über einen Zeitraum von bis zu drei Monaten verabreicht.

### BEISPIELE

### Beispiel 1: Einfluss der erfindungsgemäßen Zusammensetzung auf den Heilungsprozess nach einer Nasenoperation (Nasenscheidewandoperation)

In diesem Beispiel wird die Wirkung der erfindungsgemäßen Zusammensetzung in der Nachbetreuung einer Nasenoperation beschrieben.

Um den Einfluss verschiedenster Zusammensetzungen auf den Heilungsprozess nach einer Nasenscheidewandoperation zu untersuchen wurden insgesamt zwanzig Studienteilnehmer nach der Operation mit unterschiedlichen ölhaltigen Zusammensetzung behandelt.

Die Studienteilnehmer wurden in vier Gruppen zu je 5 Personen unterteilt, siehe Tabelle 1.

**Tabelle 1**

| **Gruppe** | **Behandlung** |
|---|---|
| 1 | Negativkontrolle |
| 2 | Zusammensetzung 1 |
| 3 | Zusammensetzung 2 |
| 4 | Zusammensetzung 3 (erfindungsgemäßes Zusammensetzung) |

Entsprechend der Zuteilung gemäß Tabelle 1, wurde die übliche Nachbehandlung der Operation (beispielsweise das Verwenden/Entfernen von Nähten und Nasentamponaden) durch die entsprechende Zusammensetzung (Gruppen 1 bis 4) ergänzt.

Die entsprechende Zusammensetzung wurde von Tag 1 nach der Operation bis zum Abschluss der Wundheilung täglich drei Mal im Abstand von 8 Stunden angewandt, wobei je ein Sprühstoß pro Nasenloch eingesetzt wurde. Da keiner der Teilnehmer Unverträglichkeiten zeigte, konnten alle Patienten bis zur vollständigen Genesung mit der entsprechenden Zusammensetzung behandelt werden.

Tabelle 2 zeigt die genaue Zusammensetzung der verwendeten Zusammensetzungen.

**Tabelle 2**

| **Inhaltsstoff [Gew. %]** | **Negativkontrolle (Gruppe 1)** | Zusammensetzung **1 (Gruppe 2)** | Zusammensetzung **2 (Gruppe 3)** | Zusammensetzung **3 (Gruppe 4)** |
|---|---|---|---|---|
| Raffiniertes Olivenöl | 100,00 | - | 96,75 | 97,15 |
| Raffiniertes Mandelöl | - | 96,00 | - | - |
| Zitronenöl | - | - | 1,05 | 0,95 |
| Pfefferminzöl | - | 2,00 | 0,85 | 0,75 |
| Eukalyptusöl | - | 2,00 | 0,70 | 0, 60 |
| Lavendelöl | - | - | 0, 60 | 0,55 |

Die Wirkung der getesteten Behandlungen der Gruppen 1 bis 4 (siehe Tabelle 1), wurde durch die benötigte durchschnittliche Zeitspanne der Wundheilung von einem Facharzt bewertet. Die Ergebnisse sind in Tabelle 3 dargestellt.

**Tabelle 3**

| **Gruppe** | **Wundheilungsdauer [Tage]** |
|---|---|
| 1 | 34, 8 |
| 2 | 31,4 |
| 3 | 23,7 |
| 4 | 18,2 |

Bei Studienteilnehmern der Gruppe 1 (raffiniertes Olivenöl) war die Wundheilungsdauer mit knapp 35 Tagen am höchsten.

Die Dauer der Wundheilung betrug bei Anwendung der Zusammensetzung 1 (Gruppe 2) knapp über 31 Tage. Allerdings wurde bei einem der fünf Teilnehmer dieser Gruppe eine leichte, klinisch irrelevante, allergische Reaktion beobachtet. Diese Reaktion scheint auf das im Zusammensetzung 1 enthaltene Mandelöl zurückzuführen sein.

Die Zusammensetzung 2 (Gruppe 3) führte zu einer Wundheilungsdauer von über 23 Tagen.

Am besten schnitt die erfindungsgemäße Zusammensetzung (Zusammensetzung 3, Gruppe 4) ab, wobei die Wundheilungsdauer auf unter 3 Wochen reduziert werden konnte. Zusätzlich wurde das Gefühl während des Heilungsprozesses von den Studienteilnehmern als angenehmer bewertet. Beispielsweise wurde der Geruch als milder empfunden, als jener der Zusammensetzung 2 (Gruppe 3). Dies ist auf die geringere Konzentration der eingesetzten ätherischen Öle zurückzuführen. Dieser verringerte Anteil der ätherischen Öle hatte jedoch keinen negativen Effekt auf die Wundheilung. Im Gegenteil, die Heilung erfolgte sogar schneller.

Beispiel 2: Einfluss der erfindungsgemäßen Zusammensetzung auf Symptome einer Rhinitis

Um den Einfluss der erfindungsgemäßen Zusammensetzung auf die Linderung der Symptome einer Rhinitis zu untersuchen, wurden vergleichbar mit Beispiel 1 20 Patienten (eingeteilt in 4 Gruppen zu je 5 Patienten), ab den zweiten Tag, an dem die ersten Symptome (Juckreiz, übermäßige Absonderung von dick- oder dünnflüssigem Sekret und Schwellung der Nasenschleimhaut und damit Behinderung der Nasenatmung) mit den Zusammensetzungen 1 bis 4 gemäß Beispiel 1 mittels Nasenspray (1 Sprühstoß pro Nasenloch, 3 Mal täglich) behandelt. Um den Heilungsverlauf zu beobachten, mussten die Patienten einen Fragebogen bzgl. der zuvor genannten Symptome beantworten. Die Patienten wurden im Sinne dieses Beispiels am ersten Tag ohne Symptome als geheilt angesehen.

Die Auswertung der Fragebögen der Patienten ergab folgende Heilungsdauer (Durchschnittswerte von jeweils 5 Patienten):

**Tabelle 4**

| **Gruppe** | **Heilungsdauer [Tage]** |
|---|---|
| 1 | 11,4 |
| 2 | 10, 2 |
| 3 | 8, 3 |
| 4 | 6, 9 |

### Beispiel 3: Einfluss der erfindungsgemäßen Zusammensetzung auf die Viabilität von Viren

Um die Wirkung der erfindungsgemäßen Zusammensetzungen auf Viren zu untersuchen werden die Zusammensetzungen 1 bis 3 (siehe Tabelle 2, Beispiel 1) und eine Negativkontrolle (siehe Tabelle 2, Beispiel 1) mit SARS-CoV-2 inkubiert, wobei bei Zusammensetzung 1 das Mandelöl mit Olivenöl ersetzt wird. Dabei werden je 0,1 ml einer SARS-CoV-2 Lösung mit einer Virenkonzentration von 10⁹ PFU/ml (PFU, "plaque forming units") mit jeweils 5 µl einer der Zusammensetzungen 1 bis 3 und der Negativkontrolle in Kontakt gebracht, kurz geschüttelt und auf ausgesäte Vero-E6-Zellen gleichmäßig aufgebracht. Nach 16 Stunden Inkubation wird der Virustiter mittles Plaque-Assay bestimmt.

Nach der Überschichtung mit einem Methylcellulose-Medium werden die Zellen 3 Tage stehen gelassen. Anschließend werden die Zellen gewaschen und mit einer Kristallviolettlösung überschichtet. Das Auftreten von Löchern (Plaques) im Zellrasen zeigt eine Infektion der Zelle. Die Plaques werden makroskopisch ausgezählt.

Bei der Auswertung der vorhandenen Virenaktivität (Anzahl der Plaques) wird die Negativkontrolle mit 100% festgelegt. Die Zusammensetzung 1 zeigt im Verhältnis zur Negativkontrolle ca. 85% der Plaques, wohingegen bei Zusammensetzung 2 ca. 30% und bei Zusammensetzung 3 ca. 3% der Plaques gezählt werden. Dies zeigt, dass die beiden Zusammensetzungen 2 und 3 in der Lage sind, SARS-CoV 2 Viren (repräsentativ für Viren mit Hüllen) zu inaktivieren.

## Patentansprüche

1. Zusammensetzung umfassend 0,50 Gew% bis 1,00 Gew% Zitronenöl, 0,30 Gew% bis 0,80 Gew% Pfefferminzöl, 0,20 Gew% bis 0,65 Gew% Eukalyptusöl, 0,10 Gew% bis 0,60 Gew% Lavendelöl und Olivenöl bis insgesamt 100 Gew% zur Verwendung bei der Behandlung von Nasenerkrankungen ausgewählt aus der Gruppe bestehend aus einer Entzündung und einer Wunde, und bei der Prävention oder Behandlung einer viralen Erkrankung, welche von Viren verursacht werden, die der Mensch über die nasalen Schleimhäute aufnimmt, wobei die Zusammensetzung nasal verabreicht wird.

2. Zusammensetzung zur Verwendung nach Anspruch 1, **dadurch gekennzeichnet, dass** das Olivenöl kaltgepresstes oder warmgepresstes Olivenöl, vorzugsweise raffiniertes Olivenöl, ist.

3. Zusammensetzung zur Verwendung nach Anspruch 1 oder 2, **dadurch gekennzeichnet, dass** das Zitronenöl ein Zitronenöl von *Citrus x limon,* das Pfefferminzöl ein Pfefferminzöl von *Mentha x piperita,* das Eukalyptusöl ein Eukalyptusöl von *Eucalyptus globulus* und/oder das Lavendelöl ein Lavendelöl von *Lavandula angustifolia, Lavandula latifolia* oder *Lavandula hybrida* ist.

4. Zusammensetzung zur Verwendung nach einem der Ansprüche 1 bis 3, **dadurch gekennzeichnet, dass** die Konzentration des Zitronenöls in der Zusammensetzung 0,60 Gew% bis 1,00 Gew%, bevorzugt 0,70 Gew% bis 1,00 Gew%, noch bevorzugter 0,80 Gew% bis 1,00 Gew%, noch bevorzugter 0,90 Gew% bis 1,00 Gew%, noch bevorzugter 0,90 Gew% bis 0,95 Gew%, insbesondere 0,95 Gew% beträgt.

5. Zusammensetzung zur Verwendung nach einem der Ansprüche 1 bis 4, **dadurch gekennzeichnet, dass** die Konzentration des Pfefferminzöls in der Zusammensetzung 0,40 Gew% bis 0,80 Gew%, bevorzugt 0,50 Gew% bis 0,80 Gew%, noch bevorzugter 0,60 Gew% bis 0,80 Gew%, noch bevorzugter 0,70 Gew% bis 0,80 Gew%, noch bevorzugter 0,70 Gew% bis 0,75 Gew%, insbesondere 0,75 Gew%, beträgt.

6. Zusammensetzung zur Verwendung nach einem der Ansprüche 1 bis 5, **dadurch gekennzeichnet, dass** die Konzentration des Eukalyptusöls in der Zusammensetzung 0,30 Gew% bis 0,65 Gew%, bevorzugt 0,40 Gew% bis 0,65 Gew%, noch bevorzugter 0,50 Gew% bis 0,65 Gew%, noch bevorzugter 0,5 Gew% bis 0,60 Gew%, insbesondere 0,5 oder 0,60 Gew%, beträgt.

7. Zusammensetzung zur Verwendung nach einem der Ansprüche 1 bis 6, **dadurch gekennzeichnet, dass** die Konzentration des Lavendelöls in der Zusammensetzung 0,20 Gew% bis 0,60 Gew%, bevorzugt 0,30 Gew% bis 0,60 Gew%, noch bevorzugter 0,40 Gew% bis 0,60 Gew%, noch bevorzugter 0,50 Gew% bis 0,60 Gew%, noch bevorzugter 0,50 Gew% bis 0,55 Gew%, insbesondere 0,55 Gew%, beträgt.

8. Zusammensetzung zur Verwendung nach einem der Ansprüche 1 bis 7, **dadurch gekennzeichnet, dass** die virale Erkrankung durch ein Virus der Familie der Coronaviridae, insbesondere humanes Coronavirus 229E, humanes Coronavirus NL63, SARS-assoziiertes Coronavirus (SARS-CoV) oder SARS-assoziiertes Coronavirus Subtyp SARS-CoV-2, besonders bevorzugt von SARS-assoziiertem Coronavirus (SARS-CoV) oder SARS-assoziiertem Coronavirus Subtyp SARS-CoV-2, und/oder der Familie der Picornaviridae, insbesondere humane Rhinoviren-1 A oder B, verursacht wird.

9. Zusammensetzung zur Verwendung nach einem der Ansprüche 1 bis 7, **dadurch gekennzeichnet, dass** die Ursache der Nasenerkrankung ausgewählt ist aus der Gruppe bestehend aus einer trockenen Umgebungsluft, einem erhöhten Naseninnendruck, einer Entzündung, bevorzugt Nasennebenhöhlenentzündung, einer Allergie, einer anderen Erkrankung mit Auswirkungen auf die Nase, bevorzugt einem grippalen Infekt, einem physischen Fremdkörper, einer äußerlichen Gewalteinwirkung und einer Operation.

10. Zusammensetzung zur Verwendung nach einem der Ansprüche 1 bis 9, **dadurch gekennzeichnet, dass** die Zusammensetzung ein- bis mehrmals täglich, bevorzugt ein- bis dreimal, vorzugsweise über einen Zeitraum von bis zu 3 Monaten, bevorzugter bis zu 2 Monaten, noch bevorzugter bis zu 1 Monat, noch bevorzugter bis zu 3 Wochen, noch bevorzugter bis zu 2 Wochen, noch bevorzugter bis zu 1 Woche, verabreicht wird.

## Claims

1. A composition comprising 0.50 wt.% to 1.00 wt.% of lemon oil, 0.30 wt.% to 0.80 wt.% of peppermint oil, 0.20 wt.% to 0.65 wt.% of eucalyptus oil, 0.10 wt.% to 0.60 wt% lavender oil and olive oil up to a total of 100 wt%, for use in the treatment of nasal conditions selected from the group consisting of an inflammation and a wound, and in the prevention or treatment of a viral disease caused by viruses that the human absorbs through the nasal mucous membranes, wherein the composition is administered nasally.

2. Composition for use according to claim 1, **characterized in that** the olive oil is cold-pressed or hot-pressed olive oil, preferably refined olive oil.

3. A composition for use according to claim 1 or 2, **characterized in that** the lemon oil is a lemon oil from *Citrus x limon,* the peppermint oil is a peppermint oil from *Mentha x piperita,* the eucalyptus oil is a eucalyptus oil from *Eucalyptus globulus,* and/or the lavender oil is a lavender oil from *Lavandula angustifolia, Lavandula latifolia,* or *Lavandula hybrida.*

4. A composition for use according to any one of claims 1 to 3, **characterized in that** the concentration of the lemon oil in the composition is 0.60 wt% to 1.00 wt%, preferably 0.70 wt% to 1.00 wt%, more preferably 0.80 wt% to 1.00 wt%, even more preferably 0.90 wt% to 1.00 wt%, and most preferably 0.90 wt% to 0.95 wt%, in particular 0.95 wt%.

5. A composition for use according to any one of claims 1 to 4, **characterized in that** the concentration of peppermint oil in the composition is 0.40 wt.% to 0.80 wt.%, preferably 0.50 wt% to 0.80 wt%, more preferably 0.60 wt% to 0.80 wt%, even more preferably 0.70 wt% to 0.80 wt%, and most preferably 0.70 wt% to 0.75 wt%, in particular 0.75 wt%.

6. A composition for use according to any one of claims 1 to 5, **characterized in that** the concentration of eucalyptus oil in the composition is 0.30 wt% to 0.65 wt%, preferably 0.40 wt% to 0.65 wt%, more preferably 0.50 wt% to 0.65 wt%, even more preferably 0.50 wt% to 0.60 wt%, and in particular 0.50 wt% or 0.60 wt%.

7. A composition for use according to any one of claims 1 to 6, **characterized in that** the concentration of lavender oil in the composition is 0.20 wt% to 0.60 wt%, preferably 0.30 wt% to 0.60 wt%, more preferably 0.40 wt% to 0.60 wt%, even more preferably 0.50 wt% to 0.60 wt%, and most preferably 0.50 wt% to 0.55 wt%, in particular 0.55 wt%.

8. A composition for use according to any one of claims 1 to 7, **characterized in that** the viral disease is caused by a virus of the Coronaviridae family, in particular human coronavirus 229E, human coronavirus NL63, SARS-associated coronavirus (SARS-CoV), or SARS-associated coronavirus subtype SARS-CoV-2, particularly preferably SARS-associated coronavirus (SARS-CoV) or SARS-associated coronavirus subtype SARS-CoV-2, and/or the Picornaviridae family, in particular human rhinovirus 1 A or B.

9. A composition for use according to any one of claims 1 to 7, **characterized in that** the cause of the nasal disorder is selected from the group consisting of dry ambient air, increased intranasal pressure, an inflammation, preferably sinusitis, an allergy, another disorder affecting the nose, preferably a cold, a physical foreign body, external trauma, and surgery.

10. A composition for use according to any one of claims 1 to 9, **characterized in that** the composition is administered once to several times per day, preferably one to three times, preferably over a period of up to 3 months, more preferably up to 2 months, even more preferably up to 1 month, even more preferably up to 3 weeks, even more preferably up to 2 weeks, and most preferably up to 1 week.

## Revendications

1. Composition comprenant 0,50 % en poids à 1,00 % en poids d'huile de citron, 0,30 % pds. à 0,80 % en poids d'huile de menthe poivrée, 0,20 % en poids à 0,65 % en poids d'huile d'eucalyptus, 0,10 % en poids à 0,60 % en poids d'huile de lavande et de l'huile d'olive jusqu'à un total de 100 % en poids, pour utilisation dans le traitement d'affections nasales choisies parmi le groupe constitué d'une inflammation et d'une plaie, et dans la prévention ou le traitement d'une affection virale causée par des virus que l'être humain contracte par les muqueuses nasales, dans lequel la composition est administrée par voie nasale.

2. Composition pour utilisation selon la revendication 1, **caractérisée en ce que** l'huile d'olive est une huile d'olive pressée à froid ou à chaud, de préférence une huile d'olive raffinée.

3. Composition pour utilisation selon la revendication 1 ou 2, **caractérisée en ce que** l'huile de citron est une huile de citron issue de *Citrus x limon,* l'huile de menthe poivrée est une huile de menthe poivrée issue de *Mentha x piperita,* l'huile d'eucalyptus est une huile d'eucalyptus issue *d'Eucalyptus globulus* et/ou l'huile de lavande est une huile de lavande issue de *Lavandula angustifolia, Lavandula latifolia* ou de *Lavandula hybrida.*

4. Composition pour utilisation selon l'une des revendications 1 à 3, **caractérisée en ce que** la concentration de l'huile de citron dans la composition est comprise entre 0,60 % en poids et 1,00 % en poids, de préférence de 0,70 % en poids à 1,00 % en poids, plus préférablement de 0,80 % en poids à 1,00 % en poids, encore plus préférablement de 0,90 % en poids à 1,00 % en poids, encore plus préférablement de 0,90 % en poids à 0,95 % en poids, et en particulier de 0,95 % en poids.

5. Composition pour utilisation selon l'une des revendications 1 à 4, **caractérisée en ce que** la concentration en huile de menthe poivrée dans la composition est comprise entre 0,40 % et 0,80 % en poids, de préférence de 0,50 % en poids à 0,80 % en poids, de manière encore plus préférée de 0,60 % en poids à 0,80 % en poids, de manière encore plus préférée de 0,70 % en poids à 0,80 % en poids, de manière encore plus préférée de 0,70 % en poids à 0,75 % en poids, en particulier 0,75 % en poids.

6. Composition pour utilisation selon l'une des revendications 1 à 5, **caractérisée en ce que** la concentration en huile d'eucalyptus dans la composition est comprise entre 0,30 % et 0,65 % en poids, de préférence de 0,40 % en poids à 0,65 % en poids, de manière encore plus préférée de 0,50 % en poids à 0,65 % en poids, de manière encore plus préférée de 0,5 % en poids à 0,60 % en poids, en particulier 0,5 % en poids ou 0,60 % en poids.

7. Composition pour utilisation selon l'une des revendications 1 à 6, **caractérisée en ce que** la concentration en huile de lavande dans la composition est comprise entre 0,20 % et 0,60 % en poids, de préférence de 0,30 % en poids à 0,60 % en poids, de préférence encore de 0,40 % en poids à 0,60 % en poids, de préférence encore de 0,50 % en poids à 0,60 % en poids, de préférence encore de 0,50 % en poids à 0,55 % en poids, en particulier 0,55 % en poids.

8. Composition pour utilisation selon l'une des revendications 1 à 7, **caractérisée en ce que** la maladie virale est provoquée par un virus de la famille des Coronaviridae, en particulier le coronavirus humain 229E, le coronavirus humain NL63, le coronavirus associé au SRAS (SARS-CoV) ou le coronavirus associé au SRAS de sous-type SARS-CoV-2, de préférence le coronavirus associé au SRAS (SARS-CoV) ou le coronavirus associé au SRAS de sous-type SARS-CoV-2, et/ou de la famille des Picornaviridae, en particulier les rhinovirus humains 1 A ou B.

9. Composition pour utilisation selon l'une des revendications 1 à 7, **caractérisée en ce que** la cause de l'affection nasale est choisie parmi le groupe constitué d'un air ambiant sec, d'une pression intranasale élevée, d'une inflammation, de préférence une sinusite, d'une allergie, d'une autre affection ayant des répercussions sur le nez, de préférence une infection grippale, d'un corps étranger physique, d'un traumatisme externe et d'une intervention chirurgicale.

10. Composition pour utilisation selon l'une des revendications 1 à 9, **caractérisée en ce que** la composition est administrée une à plusieurs fois par jour, de préférence une à trois fois, de préférence sur une période allant jusqu'à 3 mois, de préférence jusqu'à 2 mois, de préférence encore jusqu'à 1 mois, de préférence encore jusqu'à 3 semaines, de préférence encore jusqu'à 2 semaines, de préférence encore jusqu'à 1 semaine.
